# EUROPÄISCHE PATENTANMELDUNG

(11) **EP 1 818 077 A1**
(43) Veröffentlichungstag der Anmeldung: **15.08.2007**
(21) Anmeldenummer: 06002277.9
(22) Anmeldetag: 03.02.2006
(51) Int. Cl.: A61N 5/06

(54) **Vorrichtung für die Lichtbehandlung der Haut**

(71) Anmelder: WaveLight AG, 91058 Erlangen (DE)
(72) Erfinder: Vogler, Klaus, Dr., 90542 Eckental (DE); Kallert, Heiko, 91448 Emskirchen (DE)
(74) Vertreter: Katérle, Axel

(57) **Zusammenfassung**

Eine Vorrichtung für die Lichtbehandlung der Haut ist gekennzeichnet durch eine flächenhafte organische Leuchtdiodenanordnung zur Abstrahlung von Behandlungsstrahlung. Die organische Leuchtdiodenanordnung kann beispielsweise in einer flexiblen Leuchtfolie (12) enthalten sein, welche in ein körpertragbares Flächengebilde (10), etwa ein Kleidungsstück, eine Binde oder ein Pflaster, eingenäht, eingeklebt oder anderweitig integriert ist.

## Beschreibung

Die Erfindung befasst sich mit der Lichtbehandlung der Haut zu kosmetischen oder/und medizinischen Zwecken.

Es ist bekannt, dass zahlreiche Hautkrankheiten (z.B. Psoriasis, Vitiligo, Akne, atopische Dermatitis) und Hautmakel (z.B. Pigmentflecken, Narben, Warzen oder künstlich herbeigeführte Makel wie Tätowierungen) mit elektromagnetischer Strahlung wirksam behandelt werden können. Die Wirkung der elektromagnetischen Strahlung, die hier kurz mit Licht bezeichnet wird, aber nicht nur Wellenlängen im für das menschliche Auge sichtbaren Bereich, sondern auch im unsichtbaren Bereich umfassen soll, hängt dabei unter anderem von der Wellenlänge ab. Bestimmte Wellenlängen haben sich bei einzelnen Krankheiten oder Makeln als besonders wirksam erwiesen, während andere Wellenlängen bei solchen Krankheiten oder Makeln weniger wirksam oder sogar unwirksam sein können. Deswegen wird für die Lichtbehandlung der Haut in der Regel eine vergleichsweise schmalbandige oder sogar monochromatische Behandlungsstrahlung angestrebt.

Laser bilden eine häufig zur photodermatologischen Behandlung eingesetzte Strahlungsquelle. Bekanntlich haben Laser den Vorteil einer monochromatischen Emission und einer relativ hohen Strahlungsleistung und sind deshalb bei bestimmten Hautindikationen ausgesprochen wirksam, zumal da es die hohe Leistung in der Regel leicht erlaubt, die für den gewünschten kosmetischen oder Heileffekt erforderlichen Intensitäten der Behandlungsstrahlung zu erreichen. Allerdings eignen sich Laser naturgemäß vorwiegend zur Behandlung vergleichsweise kleinflächiger, insbesondere punktueller Hautbereiche. Sind dagegen größere Hautbereiche zu bestrahlen, so muss der Laserstrahl, der ja nur einen Teil dieses Hautbereichs abdeckt, nach und nach über den gesamten Hautbereich bewegt werden. Es ist leicht vorstellbar, dass dabei eine gleichmäßige Bestrahlung des gesamten Hautareals nur schwer zu erzielen ist.

Besonders für vergleichsweise großflächige photodermatologische Behandlungen wurden im Stand der Technik Lösungen vorgeschlagen, die entweder ein zum Teil aus optischen Fasern hergestelltes Gewebe oder eine Anordnung auf einem flexiblen Substrat angebrachter anorganischer Lumineszenzdioden (LED) verwenden. Ein mit optischen Fasern hergestelltes "Leuchtgewebe" ist in EP 1 147 786 A2 gezeigt. Die Fasern sind an einem aus dem Gewebe herausgeführten Ende zu einem Bündel zusammengefasst und dort mit einer Lichtquelle gekoppelt. Das in die Fasern eingespeiste Licht tritt aufgrund der zahlreichen vergleichsweise starken Biegungen jeder einzelnen Faser nach und nach aus den Fasern aus. Wegen des insgesamt ungerichteten Lichtaustritts aus den optischen Fasern ist das Leuchtgewebe auf einer Seite von einer Lage eines reflektierenden Materials unterlegt, so dass alles Licht zur selben Seite des Leuchtgewebes abgestrahlt wird. Eine auf dieser Abstrahlseite auf dem Leuchtgewebe angeordnete Streuschicht sorgt für eine Homogenisierung des abgestrahlten Lichts.

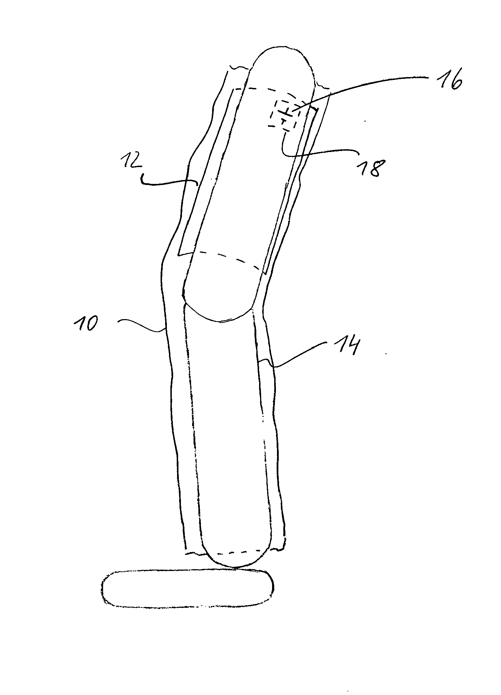

Ein Beispiel einer therapeutischen Lichtquelle mit LEDs, die auf einem flexiblen Substrat angeordnet sind, ist in US 6,645,230 beschrieben und dort in den Figuren 23a bis 23c gezeigt. Es wird in dieser Schrift die Möglichkeit angedeutet, eine solche LEDbasierte Leuchtmatte direkt auf das zu behandelnde Hautareal aufzulegen, wobei die Flexibilität des Trägersubstrats eine Anschmiegung an die Haut gestatten soll. Wegen der diskreten Natur herkömmlicher anorganischer LEDs und der nicht vermeidbaren Abstände zwischen benachbarten Dioden besteht jedoch das Problem, dass in unmittelbarer Nähe der Leuchtmatte die Strahlungsintensität ungleichmäßig sein kann, nämlich maximal direkt über den Dioden und schwächer in den Bereichen zwischen den Dioden. Erst in einem gewissen Abstand von der Leuchtmatte stellt sich infolge der Überlappung der Strahlungskegel der einzelnen Dioden eine Homogenisierung der Strahlungsintensität ein. Wird die Leuchtmatter jedoch auf die Haut aufgelegt, erfährt das betreffende Hautareal eine inhomogene Einstrahlung.

Zudem können Fertigungstoleranzen bei anorganischen Leuchtdioden zu nicht unerheblichen Schwankungen der Diodenparameter führen, mit der Folge, dass in einer Leuchtmatte, wie sie in US 6,645,230 vorgeschlagen wird, einzelne Leuchtdioden stärker strahlen können als andere. Auch dies führt zu Inhomogenitäten in der Strahlungsintensität entlang der Leuchtmatte.

Aufgabe der Erfindung ist es, eine Vorrichtung für die medizinische oder/und kosmetische Lichtbehandlung der Haut bereitzustellen, die eine homogene Bestrahlung ausgedehnter Hautregionen gestattet.

Zur Lösung dieser Aufgabe schlägt die Erfindung eine Vorrichtung für die Lichtbehandlung der Haut vor, welche eine flächenhafte organische Leuchtdiodenanordnung zur Abstrahlung von Behandlungsstrahlung aufweist. Organische Leuchtdioden (O-LED) sind aus organischen halbleitenden Polymeren oder kleinen Molekülen gebildet. Die Erfindung macht sich zunutze, dass mit OLEDs im Unterschied zu anorganischen Leuchtdioden großflächig homogen strahlende Leuchtflächen hergestellt werden können. Das für die Emission verantwortliche organische Material kann wie ein flächiger Film zwischen einer Kathodenschicht und einer Anodenschicht angeordnet werden. Deswegen sind mit OLEDs Flächenstrahler realisierbar, deren Emission über die gesamte Fläche des Strahlers äußerst homogen ist, und zwar schon in unmittelbarer Nähe zur Strahleroberfläche. Auf OLEDs basierende Bestrahlungsvorrichtungen eignen sich daher besonders gut zur Behandlung der Haut, wenn der Strahler direkt mit der Haut in Kontakt gebracht wird oder in enger räumlicher Nähe zur Haut angeordnet wird.

Vorteilhaft ist in diesem Zusammenhang, dass OLED-Strahler in Form flexibler Leuchtfolien hergestellt werden können und in solcher Form auch marktüblich erhältlich sind. Solche Leuchtfolien können dann zweckmäßigerweise in körpertragbare Flächengebilde eingearbeitet werden, nämlich derart, dass bei ordnungsgemäßem Tragen des Flächengebildes am Körper die Leuchtfolie mit einer Abstrahlseite der Körperhaut zugewandt ist. Der Begriff körpertragbare Flächengebilde soll hier jegliche flexiblen, textilen oder nichttextilen Gegenstände umfassen, die am Körper zu Bekleidungs- oder therapeutischen Zwecken getragen werden können. Beispielsweise ist es denkbar, OLED-Leuchtfolien in Hemden, Hosen, Pullover, Shirts, Mützen, Strümpfe und Unterwäscheartikel einzuarbeiten, etwa durch Einnähen oder Einkleben. Vorstellbar ist auch, Pflaster, Manschetten, Binden oder andere medizinische Artikel, die am Körper anbringbar sind, mit OLED-Leuchtfolien auszuführen. Die hohe Flexibilität bereits heute erhältlicher OLED-Leuchtfolien ermöglicht dabei eine sanfte Anschmiegung an die Haut, was der Gleichmäßigkeit der Behandlung zugute kommt. Es versteht sich, dass zum Schutz der Leuchtfolien deren Abstrahlseite von einer klaren oder matten transparenten Schutzschicht überdeckt sein kann.

Es sind im Rahmen der Erfindung selbstverständlich auch Lösungen nicht ausgeschlossen, bei denen die organische Leuchtdiodenanordnung in einem handtragbaren oder stationär aufstellbaren Lichtbehandlungsgerät angeordnet ist.

Wie bei anorganischen Leuchtdioden stehen auch bei den organischen Leuchtdioden eine Vielzahl monochromatischer Emissionswellenlängen zur Verfügung.. Damit lässt sich für verschiedenste Anwendungsfälle jeweils eine optimale Wellenlänge auswählen, beispielsweise 405 nm für Aknebehandlungen, 308 nm bei Psoriasis und Vitiligo, 640 nm für die photodynamische Therapie). Bei einer Ausführungsform ist die organische Leuchtdiodenanordnung zur Abstrahlung von Behandlungsstrahlung einer einzigen Wellenlänge ausgebildet. Gemäß einer anderen Ausführungsform dagegen kann die organische Leuchtdiodenanordnung zur Abstrahlung von Behandlungsstrahlung verschiedener Wellenlängen ausgebildet sein. Dabei ist es vorstellbar, dass die organische Leuchtdiodenanordnung wenigstens einen Teil der Wellenlängen selektiv einzeln emittieren kann, so dass beispielsweise je nach medizinischer Indikation eine andere Wellenlänge zum Einsatz gebracht werden kann. Ebenso ist es vorstellbar, dass zwei oder mehr emittierbare Wellenlängen gleichzeitig zum Einsatz bringbar sind, so dass ein multichromatisches Spektrum erzeugbar ist, das für einen breiten Anwendungsbereich geeignet sein kann.

Zur Verdeutlichung eines praktischen Anwendungsbeispiels der Erfindung wird auf die beigefügte Zeichnung verwiesen, deren einzige Figur schematisch einen Teil eines Hosenbeins 10 zeigt, in das eine OLED-Leuchtfolie 12 integriert ist. Die Leuchtfolie 12 ist mit ihrer Abstrahlseite auf die Oberfläche des zu behandelnden Beins - bezeichnet mit 14 - gerichtet. Zur elektrischen Energieversorgung der OLEDs genügt eine Niedervoltbatterie 16, die gemäß einer Variante auswechselbar in die Hose oder sogar direkt in die Leuchtfolie 12 integriert sein kann. Beispielsweise kann hierzu in der Hose oder in der Leuchtfolie eine spezielle Aufnahmetasche oder ein spezielles Aufnahmefach für die Batterie gebildet sein, wobei diese Aufnahmetasche oder dieses Aufnahmefach durch eine in die Hose oder/und in die Leuchtfolie 12 integrierte Verdrahtung mit den OLEDs verbunden sein kann. Im gezeigten Beispielfall ist die Batterie 16 in einer an der Leuchtfolie 12 gebildeten Tasche 18 untergebracht. Als Batterie kann auch eine flexible Batterie eingesetzt werden, um den Tragekomfort zu erhöhen.

Es ist sogar vorstellbar, einen körpertragbaren Artikel mit einer OLED-Leuchtfolie und einer nicht auswechselbaren Batterie auszustatten. So kann beispielsweise ein Lichtbehandlungspflaster mit integrierter Batterie bereitgestellt werden, das nur solange benutzbar ist, wie die Batterie leistungsfähig ist. Kann die Batterie keinen Strom mehr liefern, muss das Pflaster ausgetauscht werden.

Die OLEDs in der Leuchtfolie 12 können gepulst oder kontinuierlich betrieben werden. Kann die Leuchtfolie 12 verschiedene Wellenlängen emittieren, so ist es ferner denkbar, dass jede einzelne dieser Wellenlängen wahlweise zum Einsatz gebracht werden kann. Eine entsprechende Steuerschaltung für den Pulsbetrieb oder/und die Wellenlängensteuerung kann beispielsweise in die Leuchtfolie 12 integriert sein. Selbstverständlich kann auch eine von der Leuchtfolie 12 getrennte Steuereinheit vorgesehen sein, die mit der Leuchtfolie 12 geeignet verdrahtet ist. Ein in der Figur nicht näher dargestellter Schalter, beispielsweise ein Druckschalter, zum manuellen Ein- und Ausschalten des Leuchtbetriebs der Leuchtfolie 12 kann ebenfalls in die Leuchtfolie 12 oder in die Hose integriert sein.

## Patentansprüche

1. Vorrichtung für die Lichtbehandlung der Haut, **gekennzeichnet durch** eine flächenhafte organische Leuchtdiodenanordnung zur Abstrahlung von Behandlungsstrahlung.

2. Vorrichtung nach Anspruch 1, **dadurch gekennzeichnet, dass** die organische Leuchtdiodenanordnung in mindestens einer flexiblen Leuchtfolie (12) enthalten ist.

3. Vorrichtung nach Anspruch 2, **dadurch gekennzeichnet, dass** die Leuchtfolie (12) in ein körpertragbares Flächengebilde (10) eingearbeitet ist, derart, dass bei ordnungsgemäßem Tragen des Flächengebildes am Körper die Leuchtfolie mit einer Abstrahlseite der Körperhaut zugewandt ist.

4. Vorrichtung nach Anspruch 1 oder 2, **dadurch gekennzeichnet, dass** die organische Leuchtdiodenanordnung in einem handtragbaren oder stationär aufstellbaren Lichtbehandlungsgerät angeordnet ist.

5. Vorrichtung nach einem der vorhergehenden Ansprüche, **dadurch gekennzeichnet, dass** die organische Leuchtdiodenanordnung zur Abstrahlung von Behandlungsstrahlung einer einzigen Wellenlänge ausgebildet ist.

6. Vorrichtung nach einem der vorhergehenden Ansprüche, **dadurch gekennzeichnet, dass** die organische Leuchtdiodenanordnung zur Abstrahlung von Behandlungsstrahlung verschiedener Wellenlängen ausgebildet ist.

7. Verwendung einer organischen Leuchtdiodenanordnung zur Lichtbehandlung der Haut.
